# EUROPÄISCHE PATENTSCHRIFT

(11) **EP 0 790 976 B1**
(45) Veröffentlichungstag und Bekanntmachung des Hinweises auf die Patenterteilung: **21.04.1999**
(21) Anmeldenummer: 95937057.8
(22) Anmeldetag: 09.11.1995
(51) Int. Cl.: C07C 209/48, B01J 27/185, B01J 27/187, B01J 23/889

(54) **VERFAHREN ZUR HERSTELLUNG VON AMINEN AUS VERBINDUNGEN MIT MINDESTENS 3 CYANOGRUPPEN**
PROCESS FOR PREPARING AMINES FROM COMPOUNDS WITH AT LEAST 3 CYANO GROUPS
PROCEDE DE PREPARATION D'AMINES A PARTIR DE COMPOSES CONTENANT AU MOINS 3 GROUPES CYANO

(30) Priorität: 12.11.1994 DE 4440551
(43) Veröffentlichungstag der Anmeldung: 27.08.1997
(73) Patentinhaber: BASF AKTIENGESELLSCHAFT, 67056 Ludwigshafen (DE)
(72) Erfinder: HÄUSSLING, Lukas, D-67098 Bad Dürkheim (DE); NEUHAUSER, Horst, D-67373 Dudenhofen (DE); PAULUS, Wolfgang, D-67256 Weisenheim (DE)
(74) Vertreter: Isenbruck, Günter, Dr.
(86) Internationale Anmeldenummer: EP9504408
(87) Internationale Veröffentlichungsnummer: WO9615097

(56) Entgegenhaltungen:
- EP-A- 0 383 132
- EP-A- 0 636 409
- DE-A- 2 312 591
- US-A- 3 565 957
- US-A- 5 105 015
- ANGEWANDTE CHEMIE, Bd. 105, Nr. 9, 1993 Seiten 1367-72, CH. WÖRNER ET AL. 'Polynitril- und polyaminfunktionalisierte Poly(trimethylenimin)-Dendrimere' in der Anmeldung erwähnt

## Beschreibung

Die vorliegende Erfindung betrifft ein kontinuierliches Verfahren zur Herstellung von Aminen durch Hydrierung von Verbindungen mit mindestens 3 Cyanogruppen, hergestellt durch Addition von Acrylnitril an Ammoniak oder primäre Amine, bei erhöhten Temperaturen und erhöhtem Druck an neuen Kobaltkatalysatoren.

Aus der Angew. Chem. 105 (1993) 1367 bis 1372 ist die diskontinuierliche Reduktion von Nitrilen mit Wasserstoff an Raney-Kobalt in methanolischer Lösung bekannt.

Aus der DE-A-27 39 917 sind Katalysatoren zur Hydrierung von cyanethylierten Aminen bekannt, die Kobalt oder Nickel auf einem geeigneten Träger als Katalysatoren enthalten.

Die bislang bekannten diskontinuierlichen Verfahren zur Reduktion mehrfach cyanogruppenhaltiger Verbindungen sind für eine Produktion im industriellen Maßstab wirtschaftlich nicht attraktiv.

Demgemäß wurde ein neues und verbessertes Verfahren zur Herstellung von Aminen durch Hydrierung von Verbindungen mit mindestens 3 Cyanogruppen, hergestellt durch Addition von Acrylnitril an Ammoniak oder primäre Amine, bei Temperaturen von 80 bis 200°C und einem Druck von 5 bis 500 bar gefunden, welches dadurch gekennzeichnet ist, daß man die Hydrierung kontinuierlich an einem Katalysator, der 80 bis 98 Gew.-% Kobaltoxid, 1 bis 10 Gew.-% Manganoxid, 1 bis 10 Gew.-% Phosphorpentoxid und 0 bis 5 Gew.% Alkalimetalloxide enthält, durchführt sowie neue Kobaltkatalysatoren.

Die erfindungsgemäße Hydrierung läßt sich wie folgt durchführen:

Das bei der Acrylnitriladdition an Ammoniak oder primären Aminen gebildete Aminonitril, mit einer oder mehreren Nitrilgruppen, kann in An- oder Abwesenheit von Ammonaik in Gegenwart von Wasserstoff bei Temperaturen von 80 bis 200°C, bevorzugt 100 bis 160°C, besonders bevorzugt 110 bis 150°C und Drücken von 5 bis 500 bar, bevorzugt 50 bis 300 bar, besonders bevorzugt 100 bis 250 bar an einem Katalysator, der 80 bis 98 Gew.-% Kobaltoxid, 1 bis 10 Gew.-% Manganoxid und 1 bis 10 Gew.-% Phosphorpentoxid und 0 bis 5 Gew.-% Alkalimetalloxid, bevorzugt 86 bis 94 Gew.-% Kobaltoxid, 3 bis 7 Gew.-% Manganoxid und 3 bis 7 Gew.-% Phosphorpentoxid und 0 bis 5 Gew.-% Alkalimetalloxid, besonders bevorzugt 88 bis 92 Gew.-% Kobaltoxid, 4 bis 6 Gew.-% Manganoxid, 4 bis 6 Gew.-% Phosphorpentoxid und 0 bis 4 Gew.-% Alkalimetalloxid enthält, in der Regel in einem inerten Lösungsmittel umgesetzt werden.

Die zuvor genannten Kobaltkatalysatoren, ausgenommen Raney-Kobalt, können als Trägerkatalysatoren auf inerten Trägern wie Aluminiumoxid, Siliciumdioxid, Titanoxid, Zirkonoxid oder Zeolithen des Pentasil-, Faujasite-, X- oder Y-Typs oder bevorzugt als Vollkatalysatoren eingesetzt werden. Diese Kobaltkatalysatoren bestehen bevorzugt nur aus Kobaltoxid, Manganoxid, Phosphorpentoxid und Alkalimetalloxid, bevorzugt Na₂O, gegebenenfalls auf inerten Trägern. Die Herstellung der erfindungsgemäß zu verwendenden Katalysatoren ist z.B. in der DE-A 43 25 847 sowie in den Deutschen Patentanmeldungen 19516845 und 19507398 beschrieben.

Die Hydrierung kann ohne Ammoniak gefahren werden. Bevorzugt wird aber Ammoniak in der Regel gasförmig oder flüssig, im allgemeinen jedoch nicht in wäßriger Lösung, im Verhältnis von Mol Ammoniak pro Cyanogruppe des intermediär gebildeten Aminonitrils von 1:1 bis 10000:1, bevorzugt 1,5:1 bis 3000:1, besonders bevorzugt 2:1 bis 1000:1 eingesetzt. Der Ammoniaküberschuß kann ohne weiteres auch größer als 10000:1 sein.

Der Wasserstoff wird der Reaktion im allgemeinen in einer Menge von 5 bis 400 Nl, bevorzugt in einer Menge von 50 bis 200 Nl pro Mol Aminonitril zugeführt.

Praktisch geht man bei der Durchführung der Umsetzung so vor, daß man dem Heterogenkatalysator, der sich üblicherweise in einem bevorzugt von außen beheizten Festbettreaktor befindet, bei der gewünschten Reaktionstemperatur und dem gewünschten Druck das Aminonitril in einem inerten Lösungsmittel und Ammoniak simultan zuführt. Dabei belastet man den Kobaltkatalysator im allgemeinen mit 0,001 bis 5,0, bevorzugt 0,005 bis 2,0 und insbesondere bevorzugt mit 0,01 bis 1,5 l Aminonitril pro Liter Kobaltkatalysator und Stunde.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten.

Die Reaktion wird kontinuierlich durchgeführt. Der überschüssige Ammoniak kann zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag können, nachdem der Druck zweckmäßigerweise reduziert worden ist, der überschüssige Ammoniak und der Wasserstoff entfernt und das erhaltene Produkt - sofern gewünscht - z.B. durch Destillation oder Extraktion gereinigt werden. Ammoniak und Wasserstoff können vorteilhaft wieder in die Reaktionszone zurückgeführt werden. Das gleiche gilt für das eventuell nicht vollständig umgesetzte Aminonitril.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und kann deshalb zweckmäßigerweise erst bei der destillativen Aufarbeitung des Reaktionsproduktes aus diesem entfernt werden.

Als inerte Lösungsmittel eignen sich Alkohole z.B. C₁- bis C₂₀-Alkanole, bevorzugt C₁- bis C₈-Alkanole, besonders bevorzugt C₁- bis C₄-Alkanole wie Methanol und Ethanol, Ether z.B. aliphatische C₄- bis C₂₀-Ether, bevorzugt C₄- bis C₁₂-Ether wie Diethylether, Methyl-iso-propylether und Methyl-tert.-butylether, cyclische Ether wie Tetrahydrofuran und Dioxan, Glykolether wie Ethylenglykoldimethylether, Dimethylformamid, gesättigte Stickstoffheterocyclen mit tertiärem Stickstoff wie N-Methylpyrrolidon.

Als primäre Amine für die Acrylnitril-Addition und die anschließende erfindungsgemäße Hydrierung eignen sich solche der allgemeinen Formel I

(R¹R¹)N - X - N(R¹R¹) (I),

in der R¹ Wasserstoff oder (R²R²)N-(CH₂)₃- und R² Wasserstoff oder (R³R³)N-(CH₂)₃- und R³ Wasserstoff oder (R⁴R⁴)N-(CH₂)₃- und R⁴ Wasserstoff oder (R⁵R⁵)N-(CH₂)₃- und R⁵ Wasserstoff oder (R⁶R⁶)N-(CH₂)₃- und R⁶ Wasserstoff bedeuten, und
- X: für 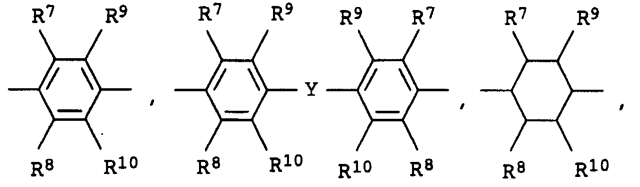 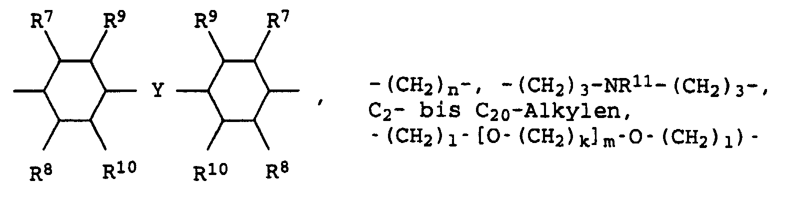 und Y für CR⁷R⁹, Sauerstoff, C=O, SO₂, n für 2 bis 20, l und k für 2 bis 6 und m für 1 bis 40,
- R⁷, R⁸, R⁹, R¹⁰: für Wasserstoff oder C₁- bis C₆-Alkyl,
- R¹¹: für C₁- bis C₂₀-Alkyl, C₂-bis C₂₀-Dialkylamino-C₂- bis C₁₀-alkyl, C₁-bis C₁₀-Alkoxy-C₂ -bis C₁₀-alkyl, C₂- bis C₂₀-Hydroxyalkyl, C₃- bis C₁₂-Cycloalkyl, C₄- bis C₂₀-Cycloalkyl-alkyl, C₂- bis C₂₀-Alkenyl, C₄- bis C₃₀-Dialkylaminoalkenyl, C₃- bis C₃₀-Alkoxy-alkenyl, C₃- bis C₂₀-Hydroxyalkenyl, C₅- bis C₂₀-Cycloalkyl-alkenyl, gegebenenfalls durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl, ein- bis fünffach substituiertes Aryl oder C₇-bis C₂₀-Aralkyl oder gemeinsam eine gegebenenfalls durch Stickstoff oder Sauerstoff unterbrochene Alkylenkette wie Ethylenoxid, Propylenoxid, Butylenoxid und -CH₂-CH(CH₃)-O- oder Polyisobutylen mit 1 bis 100 iso-Butyleneinheiten
stehen, bevorzugt solche der Formel II

NH₂-(CH₂)ₙ-NH₂ (II),

in der n für 2 bis 20 steht.

Diamine der Formel II sind bevorzugt primäre Diamine wie 1,2-Ethylendiamin, 1,3-Propylendiamin, 1,4-Butylendiamin,und 1,6-Hexamethylendiamin, bevorzugt primäre Tetraamine wie N,N,N',N'-Tetraaminopropyl-1,2-ethylendiamin, N,N,N',N'-Tetraaminopropyl-1,3-propylendiamin, N,N,N',N'-Tetraaminopropyl-1,4-butylendiamin und N,N,N',N'-Tetraaminopropyl-1,6-hexamethylendiamin, bevorzugt primäre Octamine sowie Ammoniak.

Die Reste R⁷, R⁸, R⁹, R¹⁰ bedeuten C₁- bis C₆-Alkyl, bevorzugt C₁-bis C₃-Alkyl wie Methyl, Ethyl, n-Propyl und iso-Propyl, besonders bevorzugt Methyl und Ethyl, insbesondere Methyl oder bevorzugt Wasserstoff, wobei R⁷ und R⁸ bzw. R⁹ und R¹⁰ bevorzugt gleich sind.

Im einzelnen bedeutet der Rest R¹¹ C₁- bis C₂₀-Alkyl, bevorzugt C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl, Aryl wie Phenyl, 1-Naphthyl und 2-Naphthyl, bevorzugt Phenyl, C₇- bis C₂₀-Aralkyl, bevorzugt C₇- bis C₁₂-Phenylalkyl wie Benzyl, 1-Phenethyl, 2-Phenethyl, 1-Phenylpropyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl, C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl oder Polyisobutylen mit 1 bis 100, bevorzugt 1 bis 70, besonders bevorzugt 1 bis 50 iso-Butyleneinheiten

Die erfindungsgemäßen Amine eignen sich als Verzweigungseinheiten für Nylon 6 [Chem. Mater. 4 (5) 1000 bis 1004 (1992)], als Diagnostika und Kontrastmittel in der Computertomographie [Chem. Mark. Rep. 28. Dec. 1992, 242 (26), 19], als Standard in der Gelpermeationschromatographie, oder in der Kosmetik und Pharmaindustrie als controlled released Verbindungen.

### Beispiele

Die Produkte in den folgenden Beispielen wurden mittels ¹³C⁻ und ¹H-NHR- sowie Massenspektroskopie auf Einheitlichkeit und Vollständigkeit der Reaktion überprüft.

### Beispiel 1

Zu einer intensiv gerührten Lösung aus 100 g (1,67 mol) Ethylendiamin in 1176 ml Wasser wurde bei maximal 40°C innerhalb 90 min 443 g (8,35 mol) Acrylnitril zugetropft, 1 h bei Raumtemperatur und 2 h bei 80°C gerührt und anschließend i. Vak. bei maximal 80°C eingeengt und der feste Rückstand 478 g (1,58 mol), das N,N,N',N'-Tetracyanoethyl-1,2-ethylendiamin, abfiltriert.

Man leitete 400 ml/h eines Gemisches aus 20 Gew.-% N,N,N',N'-Tetracyanoethyl-1,2-ethylendiamin und 80 Gew.-% N-Methyl-pyrrolidon und 3500 ml/h Ammoniak bei 130°C und 200 bar Wasserstoffdruck in einem 5 Liter Festbettreaktor über 4 Liter eines Festbettkatalysators der Zusammensetzung 90 Gew.-% CoO, 5 Gew.-% MnO und 5 Gew.-% P₂O₅. Nach Entfernung des Lösungsmittels i. Vak. und fraktionierender Destillation Sdp.: 218°C (6 mbar), erhielt man N,N,N',N'-Tetraaminopropyl-1,2-ethylendiamin mit einer Ausbeute von 95 %.

### Vergleichsbeispiel (nach Angew. Chem. 105 (1993) 1367)

Zu einer Lösung aus 400 ml Alkohol-Wasser im Volumenverhältnis von 1:1 und 1 ml 1n NaOH-Lösung wurde 20 g N,N,N',N'-Tetracyanoethyl-1,2-ethylendiamin und 50 g Raney-Nickel (wäßrige Suspension) unter Stickstoffatmosphäre in einen Rührautoklaven gegeben. Unter 8 bar Wasserstoffdruck wurde diese Mischung bei Raumtemperatur (25°C) ca. 10 Stunden hydriert.

Nach Abschluß der Hydrierung wurde der Katalysator abfiltriert, mit einer Ethanol-Wasser-Mischung gewaschen und das Filtrat destilliert. Bei einem Umsatz von 98 % ergab sich eine Selektivität von 22 %.

### Beispiel 2

Mit 300 ml/h wurde ein Gemisch aus 10 Gew.-% N,N,N',N',N",N", N‴,N‴-Octacyanoethyl-N'''',N'''',N''''',N'''''-tetraaminopropyl-1,2-ethylendiamin und 90 Gew.-% Methanol und 3000 ml/h Ammoniak bei 130°C und 200 bar Wasserstoffdruck in einen 5 Liter Festbettreaktor über 4 Liter eines Festbettkatalysators der Zusammensetzung 90 Gew.-% CoO, 5 Gew.-% MnO, 5 Gew.-% P₂O₅ geleitet. Nach Entfernung des überschüssigen Ammoniaks und des Methanols erhielt man quantitativ N,N,N',N',N", N",N"',N‴-Octaaminogropyl-N'''',N'''',N''''',N'''''-tetraaminopropyl-1,2-ethylendiamin (N14-Amin).

### Beispiel 3

Analog Beispiel 2 wurde ein Gemisch aus 10 Gew.-% N,N,N',N',N",N",N‴,N‴-Octacyanoethyl-N'''',N'''',N''''',N'''''-tetraaminopropyl-1,2-ethylendiamin und 90 Gew.-% N-Methylpyrrolidon und 3000 ml/h Ammoniak umgesetzt.

Nach Entfernung des N-Methylpyrrolidons erhielt man quantitativ N,N,N',N',N",N",N‴,N‴-Octaaminopropyl-N'''',N'''',N''''',N'''''-tetraaminopropyl-1,2-ethylendiamin (N14-Amin).

### Beispiel 4

Analog Beispiel 2 wurde ein Festbettkatalysator der ZusammenSetzung 89,6 Gew.-% CoO, 5 Gew.-% MnO, 5 Gew.-% P₂O₅ und 0,4 Gew.-% Na₂O eingesetzt. Man erhielt quantitativ N,N,N',N',N",N",N''',N'''-OctaaminopropylN'''',N'''',N''''',N'''''-tetraaminopropyl-1,2-ethylendiamin (N14-Amin).

### Beispiel 5

Analog Beispiel 3 wurde ein Festbettkatalysator der Zusammensetzung 89,6 Gew.-% CoO, 5 Gew.-% MnO, 5 Gew.-% P₂O₅ und 0,4 Gew.-% Na₂O eingesetzt. Man erhielt quantitativ N,N,N',N',N",N",N''',N'''-Octaaminopropyl-N'''',N'''',N''''',N'''''-tetraaminopropyl-1,2-ethylendiamin (N14-Amin).

### Beispiel 6

Analog Beispiel 2 wurde 50 ml eines Gemisches aus 10 Gew.-% N,N,N',N',N'',N'',N''',N'''-Octacyanoethyl-N'''',N'''',N''''',N'''''-tetraaminopropyl-1,2-ethylendiamin und 90 Gew.-% N-Methylpyrrolidon und 500 ml/h Ammoniak in einem 1 Liter Festbettreaktor an 500 ml eines Festbettkatalysators der Zusammensetzung 89,6 Gew.-% CoO, 5 Gew.-% MnO, 5 Gew.-% P₂O₅ und 0,4 Gew.-% Na₂O umgesetzt. Nach Entfernung des N-Methylpyrrolidons erhielt man quantitativ N,N,N',N',N'',N'',N''', N'''-Octaaminopropyl-N'''',N'''',N''''',N'''''-tetraaminopropyl-1,2-ethylendiamin (N14-Amin).

### Beispiel 7

Analog Beispiel 2 wurde ein Gemisch aus 13 Gew.-% hexadecacyanoethyliertes N14-Amin und 87 Gew.-% N-Methylpyrrolidon und 3000 ml/h Ammoniak an einem Festbettkatalysator der Zusammensetzung 89,6 Gew.-% CoO, 5 Gew.-% MnO, 5 Gew.-% P₂O₅ und 0,4 Gew.-% Na₂O umgesetzt. Nach Entfernung des N-Methylpyrrolidons erhielt man quantitativ hexadecaaminopropyliertes N14-Amin (N30-Amin).

### Beispiel 8

Analog Beispiel 2 wurde ein Gemisch aus 13 Gew.-% hexadecacyanoethyliertes N14-Amin und 87 Gew.-% N-Methylpyrrolidon und 3000 ml/h Ammoniak umgesetzt. Nach Entfernung des N-Methylpyrrolidons erhielt man quantitativ hexadecaaminopropyliertes N14-Amin (N30-Amin).

### Beispiel 9

Mit 50 ml/h wurde ein Gemisch aus 20 Gew.-% hexadecacyanoethyliertes N14-Amin und 80 Gew.-% N-Methylpyrrolidon und 500 ml/h Ammoniak bei 130°C und 200 bar Wasserstoffdruck in einem 1 Liter Festbettreaktor über 500 ml eines Festbettkatalysators der Zusammensetzung 89,6 Gew.-% CoO, 5 Gew.-% MnO, 5 Gew.-% P₂O₅ und 0,4 Gew.-% Na₂O geleitet. Nach Entfernung des N-Methylpyrolidons erhielt man quantitativ hexadecaminopropyliertes N14-Amin (N30-Amin).

### Beispiel 10

Analog Beispiel 9 wurde ein Gemisch aus 10 Gew.-% hexadecacyanoethyliertes N14-Amin und 90 Gew.-% N-Methylpyrrolidon und 500 ml/h Ammoniak umgesetzt. Nach Entfernung des N-Methylpyrrolidons erhielt man quantitativ hexadecaaminopropyliertes N14-Amin (N30-Amin).

### Beispiel 11

Analog Beispiel 9 wurde ein Gemisch aus 13 Gew.-% hexadecacyanoethyliertes N14-Amin und 87 Gew.-% N-Methylpyrrolidon und 1000 ml/h Ammoniak umgesetzt. Man erhielt quantitativ hexadecaaminopropyliertes N14-Amin (N30-Amin). Man erhielt quantitativ hexadecaaminopropyliertes N14-Amin (N30-Amin).

### Beispiel 12

Analog Beispiel 9 wurde ein Gemisch aus 13 Gew.-% hexadecacyanoethyliertes N14-Amin und 87 Gew.-% N-Methylpyrrolidon und 1000 ml/h Ammoniak mit einem Festbettkatalysator der Zusammensetzung 90 Gew.-% CoO, 5 Gew.-% MnO und 5 Gew.-% P₂O₅ umgesetzt. Man erhielt quantitativ hexadecaaminopropyliertes N14-Amin (N30-Amin).

## Patentansprüche

1. Verfahren zur Herstellung von Aminen durch Hydrierung von Verbindungen mit mindestens 3 Cyanogruppen, hergestellt durch Addition von Acrylnitril an Ammoniak oder primäre Amine, bei Temperaturen von 80 bis 200°C und einem Druck von 5 bis 500 bar, dadurch gekennzeichnet, daß man die Hydrierung kontinuierlich an einem Katalysator, der 80 bis 98 Gew.-% Kobaltoxid, 1 bis 10 Gew.-% Manganoxid, 1 bis 10 Gew.-% Phosphorpentoxid und 0 bis 5 Gew.-% Alkalimetalloxide enthält, durchführt.

2. Verfahren zur Herstellung von Aminopropylaminen nach Anspruch 1, dadurch gekennzeichnet, daß man die Hydrierung an einem Katalysator, der 86 bis 94 Gew.-% Kobaltoxid, 3 bis 7 Gew.-% Manganoxid und 3 bis 7 Gew.-% Phosphorpentoxid und 0 bis 5 Gew.-% Alkalimetalloxid enthält, durchführt.

3. Verfahren zur Herstellung von Aminopropylaminen nach Anspruch 1, dadurch gekennzeichnet, daß man die Hydrierung an einem Katalysator, der 88 bis 92 Gew.-% Kobaltoxid, 4 bis 6 Gew.-% Manganoxid und 4 bis 6 Gew.-% Phosphorpentoxid und 0 bis 4 Gew.-% Alkalimetalloxid enthält, durchführt.

4. Verfahren zur Herstellung von Aminopropylaminen nach Anspruch 1, dadurch gekennzeichnet, daß man als primäre Amine solche der allgemeinen Formel I
(R¹R¹)N - X - N(R¹R¹) (I),
in der R¹ Wasserstoff oder (R²R²)N-(CH₂)₃- und R² Wasserstoff oder (R³R³)N-(CH₂)₃- und R³ Wasserstoff oder (R⁴R⁴)N-(CH₂)₃- und R⁴ Wasserstoff oder (R⁵R⁵)N-(CH₂)₃- und R⁵ Wasserstoff oder (R⁶R⁶)N-(CH₂)₃- und R⁶ Wasserstoff bedeuten,
X für 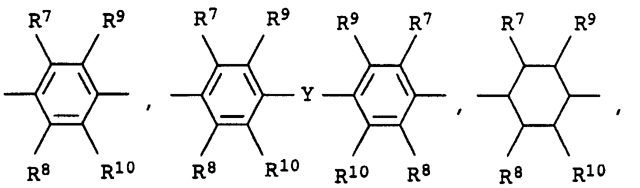 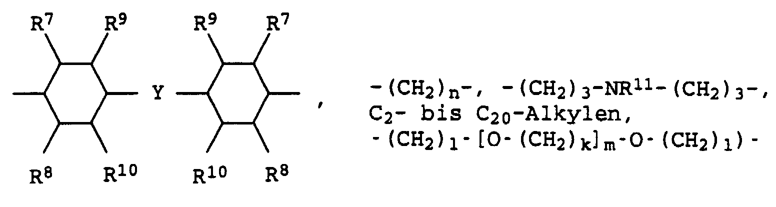 und Y für CR⁷R⁹, Sauerstoff, C=O, SO₂, n für 2 bis 20, l und k für 2 bis 6 und m für 1 bis 40,
R⁷, R⁸, R⁹, R¹⁰ für Wasserstoff oder C₁- bis C₆-Alkyl,
R¹¹ für C₁- bis C₂₀-Alkyl, C₂-bis C₂₀-Dialkylamino-C₂- bis C₁₀-alkyl, C₁-bis C₁₀-Alkoxy-C₂ -bis C₁₀-alkyl, C₂- bis C₂₀-Hydroxyalkyl, C₃- bis C₁₂-Cycloalkyl, C₄- bis C₂₀-Cycloalkyl-alkyl, C₂- bis C₂₀-Alkenyl, C₄- bis C₃₀-Dialkylamino-alkenyl, C₃- bis C₃₀-Alkoxy-alkenyl, C₃-bis C₂₀-Hydroxyalkenyl, C₅- bis C₂₀-Cycloalkyl-alkenyl, gegebenenfalls durch C₁- bis C₈-Alkyl, C₂- bis C₈-Dialkylamino, C₁- bis C₈-Alkoxy, Hydroxy, C₃- bis C₈-Cycloalkyl, C₄- bis C₁₂-Cycloalkyl-alkyl, ein- bis fünffach substituiertes Aryl oder C₇- bis C₂₀-Aralkyl oder gemeinsam eine gegebenenfalls durch Stickstoff oder Sauerstoff unterbrochene Alkylenkette wie Ethylenoxid, Propylenoxid, Butylenoxid und -CH₂-CH(CH₃)-O- oder Polyisobutylen mit 1 bis 100 iso-Butyleneinheiten stehen,
einsetzt.

5. Verfahren zur Herstellung von Aminopropylaminen nach Anspruch 1, dadurch gekennzeichnet, daß man als primäre Amine solche der Formel II
NH₂-(CH₂)ₙ-NH₂ (II),
in der n für 2 bis 20 steht, einsetzt.

6. Verfahren zur Herstellung von Aminopropylaminen nach Anspruch 1, dadurch gekennzeichnet, daß man als primäre Amine N,N,N',N'-Tetraamino-propyl-1,2-ethylendiamin, N,N,N',N'-Tetraaminopropyl-1,3-propylendiamin, N,N,N',N'-Tetraaminopropyl-1,4-butylendiamin oder N,N,N',N'-Tetraaminopropyl-1,6-hexamethylendiamin einsetzt.

7. Verfahren zur Herstellung von Aminopropylaminen nach Anspruch 1, dadurch gekennzeichnet, daß man die Hydrierung bei Temperaturen von 100 bis 160°C und Drücken von 50 bis 300 bar durchführt.

8. Kobaltkatalysator enthaltend 80 bis 98 Gew.-% Kobaltoxid, 1 bis 10 Gew.-% Manganoxid, 1 bis 10 Gew.-% Phosphorpentoxid und 0 bis 5 Gew.-% Alkalimetalloxid.

9. Kobaltkatalysator enthaltend 86 bis 94 Gew.-% Kobaltoxid, 3 bis 7 Gew.-% Manganoxid, 3 bis 7 Gew.-% Phosphorpentoxid und 0 bis 5 Gew.-% Alkalimetalloxid.

10. Kobaltkatalysator enthaltend 88 bis 92 Gew.-% Kobaltoxid, 4 bis 6 Gew.-% Manganoxid, 4 bis 6 Gew.-% Phosphorpentoxid und 0 bis 4 Gew.-% Alkalimetalloxid.

## Claims

1. A process for preparing amines by hydrogenation of compounds having at least 3 cyano groups, prepared by addition of acrylonitrile onto ammonia or primary amines, at from 80 to 200°C under from 5 to 500 bar, which comprises carrying out the hydrogenation continuously on a catalyst which contains from 80 to 98% by weight of cobalt oxide, from 1 to 10% by weight of manganese oxide, from 1 to 10% by weight of phosphorus pentoxide and from 0 to 5% by weight of alkali metal oxides.

2. A process for preparing amines as claimed in claim 1, wherein the hydrogenation is carried out on a catalyst which contains from 86 to 94% by weight of cobalt oxide, from 3 to 7% by weight of manganese oxide and from 3 to 7% by weight of phosphorus pentoxide and from 0 to 5% by weight of alkali metal oxide.

3. A process for preparing amines as claimed in claim 1, wherein the hydrogenation is carried out on a catalyst which contains from 88 to 92% by weight of cobalt oxide, from 4 to 6% by weight of manganese oxide and from 4 to 6% by weight of phosphorus pentoxide and from 0 to 4% by weight of alkali metal oxide.

4. A process for preparing amines as claimed in claim 1, wherein the primary amines used are those of the general formula I
(R¹R¹)N - X - N(R¹R¹) (I),
where R¹ is hydrogen or (R²R²)N-(CH₂)₃- and R² is hydrogen or (R³R³)N-(CH₂)₃- and R³ is hydrogen or (R⁴R⁴)N-(CH₂)₃- and R⁴ is hydrogen or (R⁵R⁵)N-(CH₂)₃- and R⁵ is hydrogen or (R⁶R⁶)N-(CH₂)₃- and R⁶ is hydrogen,
X is 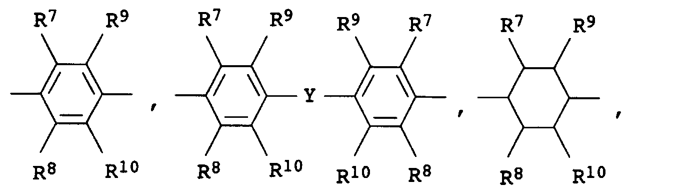 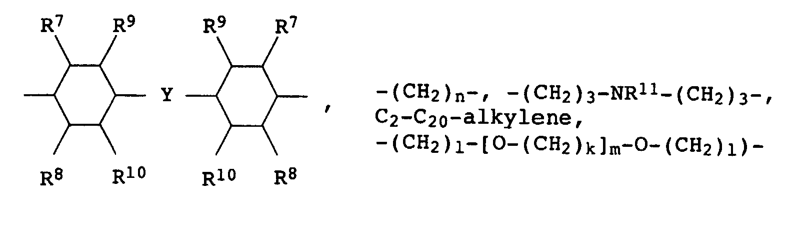 and Y is CR⁷R⁹, oxygen, C=O, SO₂, n is 2-20, l and k are 2-6 and m is 1-40,
R⁷, R⁸, R⁹, R¹⁰ are hydrogen or C₁-C₆-alkyl,
R¹¹ is C₁-C₂₀-alkyl, C₂-C₂₀-dialkylamino-C₂-C₁₀-alkyl, C₁-C₁₀-alkoxy-C₂-C₁₀-alkyl, C₂-C₂₀-hydroxyalkyl, C₃-C₁₂-cycloalkyl, C₄-C₂₀-cycloalkylalkyl, C₂-C₂₀-alkenyl, C₄-C₃₀-dialkylaminoalkenyl, C₃-C₃₀-alkoxyalkenyl, C₃-C₂₀-hydroxyalkenyl, C₅-C₂₀-cycloalkylalkenyl, or aryl or C₇-C₂₀-aralkyl which is unsubstituted or substituted once to five times by C₁-C₈-alkyl, C₂-C₈-dialkylamino, C₁-C₈-alkoxy, hydroxyl, C₃-C₈-cycloalkyl, C₄-C₁₂-cycloalkylalkyl, or together an alkylene chain which may be interrupted by nitrogen or oxygen, such as ethylene oxide, propylene oxide, butylene oxide and -CH₂-CH(CH₃)-O- or polyisobutylene with 1-100 isobutylene units.

5. A process for preparing amines as claimed in claim 1, wherein the primary amines used are those of the formula II
NH₂-(CH₂)ₙ-NH₂ (II),
where n is 2-20.

6. A process for preparing amines as claimed in claim 1, wherein N,N,N',N'-tetraaminopropyl-1,2- ethylenediamine, N,N,N',N'-tetraaminopropyl-1,3-propylenediamine, N,N,N',N'-tetraaminopropyl-1,4-butylenediamine or N,N,N',N'-tetraaminopropyl-1,6-hexamethylenediamine is used as primary amine.

7. A process for preparing amines as claimed in claim 1, wherein the hydrogenation is carried out at from 100 to 160°C under from 50 to 300 bar.

8. A cobalt catalyst containing from 80 to 98% by weight of cobalt oxide, from 1 to 10% by weight of manganese oxide, from 1 to 10% by weight of phosphorus pentoxide and from 0 to 5% by weight of alkali metal oxide.

9. A cobalt catalyst containing from 86 to 94% by weight of cobalt oxide, from 3 to 7% by weight of manganese oxide, from 3 to 7% by weight of phosphorus pentoxide and from 0 to 5% by weight of alkali metal oxide.

10. A cobalt catalyst containing from 88 to 92% by weight of cobalt oxide, from 4 to 6% by weight of manganese oxide, from 4 to 6% by weight of phosphorus pentoxide and from 0 to 4% by weight of alkali metal oxide.

## Revendications

1. Procédé de préparation d'amines par hydrogénation de composés comportant au moins trois groupes cyano, préparés par addition d'acrylonitrile sur de l'ammoniac ou des amines primaires, à des températures de 80 à 200°C et à une pression de 5 à 500 bar, caractérisé en ce qu'on effectue l'hydrogénation en continu sur un catalyseur, qui contient 80 à 98 % en poids d'oxyde de cobalt, 1 à 10 % en poids d'oxyde de manganèse, 1 à 10 % en poids de pentoxyde de phosphore et 0 à 5 % en poids d'oxydes de métal alcalin.

2. Procédé de préparation d'aminopropylamines suivant la revendication 1, caractérisé en ce qu'on effectue l'hydrogénation sur un catalyseur, qui contient 86 à 94 % en poids d'oxyde de cobalt, 3 à 7 % en poids d'oxyde de manganèse et 3 à 7 % en poids de pentoxyde de phosphore, et 0 à 5 % en poids d'oxyde de métal alcalin.

3. Procédé de préparation d'aminopropylamines suivant la revendication 1, caractérisé en ce qu'on effectue l'hydrogénation sur un catalyseur, qui contient 88 à 92 % en poids d'oxyde de cobalt, 4 à 6 % en poids d'oxyde de manganèse et 4 à 6 % en poids de pentoxyde de phosphore, et 0 à 4 % en poids d'oxyde de métal alcalin.

4. Procédé de préparation d'aminopropylamines suivant la revendication 1, caractérisé en ce qu'on met en oeuvre, comme amines primaires, celles de la formule générale I
(R¹R¹)N - X - N(R¹R¹) (I),
dans laquelle R¹ représente de l'hydrogène ou (R²R²)N-(CH₂)₃- et R² représente de l'hydrogène ou (R³R³)N-(CH₂)₃-, et R³ représente de l'hydrogène ou (R⁴R⁴)N-(CH₂)₃-, et R⁴ représente de l'hydrogène ou (R⁵R⁵)N-(CH₂)₃ -, et R⁵ représente de l'hydrogène ou (R⁶R⁶)N-(CH₂)₃- et R⁶ représente de l'hydrogène,
X représente 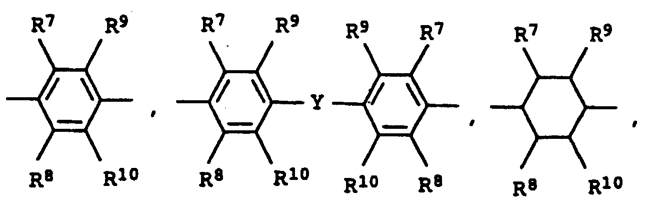 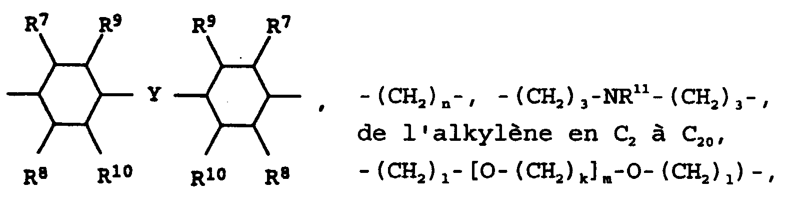 et Y représente CR⁷R⁹, de l'oxygène, C=O, SO₂, n étant égal à 2 jusqu'à 20, l et k à 2 jusqu'à 6 et m à 1 jusqu'à 40,
R⁷, R⁸, R⁹, R¹⁰ représentent de l'hydrogène ou de l'alkyle en C₁ à C₆,
R¹¹ représente de l'alkyle en C₁ à C₂₀, du C₂ à C₂₀-dialkylamino-C₂ à C₁₀-alkyle, du C₁ à C₁₀-alcoxy- C₂ à C₁₀-alkyle, de l'hydroxyalkyle en C₂ à C₂₀,du cycloalkyle en C₃ à C₁₂, du cycloalkyle-alkyle en C₄ à C₂₀, de l'alcényle en C₂ à C₂₀, du dialkylamino-alcényle en C₄ à C₃₀, de l'alcoxyalcényle en C₃ à C₃₀, de l'hydroxy-alcényle en C₃ à C₂₀, du cycloalkyl-alcényle en C₅ à C₂₀, de l'aryle éventuellement substitué à une jusqu'à cinq reprises par de l'akyle en C₁ à C₈, du dialkylamino en C₂ à C₈, de l'alcoxy en C₁ à C₈, de l'hydroxy, du cycloalkyle en C₃ à C₈, du cycloalkyl-alkyle en C₄ à C₁₂, ou de l'aralkyle en C₇ à C₂₀, ou éventuellement une chaîne alkylène éventuellement interrompue par de l'azote ou de l'oxygène, comme de l'oxyde d'éthylène, de l'oxyde de propylène, de l'oxyde de butylène et du -CH₂-CH(CH₃)-O- ou du poly-isobutylène avec 1 à 100 unités iso-butylène.

5. Procédé de préparation d'aminopropylamines suivant la revendication 1, caractérisé en ce que, comme amines primaires, on met en oeuvre celles de la formule II
NH₂-(CH₂)ₙ-NH₂ (II),
dans laquelle n est égal à 2 jusqu'à 20.

6. Procédé de préparation d'aminopropylamines suivant la revendication 1, caractérisé en ce que, comme amines primaires, on met en oeuvre de la N,N,N',N'-tétraamino-propyl-1,2-éthylène-diamine, de la N,N,N',N'-tétraaminopropyl-1,3-propylène-diamine, de la N,N,N',N'-tétraaminopropyl-1,4-butylène-diamine, ou de la N,N,N',N'-tétraaminopropyl-l,6-hexaméthylène- diamine.

7. Procédé de préparation d'aminopropylamines suivant la revendication 1, caractérisé en ce qu'on effectue l'hydrogénation à des températures de 100 à 160°C et à des pressions de 50 à 300 bar.

8. Catalyseur à base de cobalt contenant 80 à 98 % en poids d'oxyde de cobalt, 1 à 10 % en poids d'oxyde de manganèse, 1 à 10 % en poids de pentoxyde de phosphore et 0 à 5 % en poids d'oxyde de métal alcalin.

9. Catalyseur à base de cobalt contenant 86 à 94 % en poids d'oxyde de cobalt, 3 à 7 % en poids d'oxyde de manganèse, 3 à 7 % en poids de pentoxyde de phosphore et 0 à 5 % en poids d'oxyde de métal alcalin.

10. Catalyseur à base de cobalt contenant 88 à 92 % en poids d'oxyde de cobalt, 4 à 6 % en poids d'oxyde de manganèse, 4 à 6 % en poids de pentoxyde de phosphore et 0 à 4 % en poids d'oxyde de métal alcalin.
